# EUROPEAN PATENT APPLICATION

(11) **EP 1 108 426 A2**
(43) Date of publication of application: **20.06.2001**
(21) Application number: 00310511.1
(22) Date of filing: 27.11.2000
(51) Int. Cl.: A61K 31/00, A61K 31/19, A61K 31/195, A61K 31/381, A61K 31/41, A61K 31/497, A61K 31/4406, A61K 31/506, A61K 31/443, A61K 31/4433, A61K 31/4436, A61K 31/4439, A61K 31/444, A61P 15/10

(54) **Use of prostaglandin agonists to treat erectile dysfunction or impotence**

(30) Priority: 02.12.1999 US 168519 P
(71) Applicant: Pfizer Products Inc., Groton, Connecticut 06340 (US)
(72) Inventor: Cameron, Kimberley O'Keefe, Groton, Connecticut 06340 (US); Lefker, Bruce Allen, Groton, Connecticut 06340 (US)
(74) Representative: Sewell, Richard Charles

(57) **Abstract**

The present invention relates to methods of treating impotence or erectile dysfunction which comprise administering to a patient in need of such treatment an impotence or erectile dysfunction treating amount of a prostaglandin receptor agonist.

## Description

### BACKGROUND OF THE INVENTION

The present invention provides methods for treating impotence or erectile dysfunction in human male patients.

Impotence can be defined literally as a lack of power, in the male, to copulate and may involve an inability to achieve penile erection and/or ejaculation. More specifically, erectile impotence or dysfunction may be defined as an inability to obtain or sustain an erection adequate for intercourse. Its prevalence is claimed to be between two and seven percent of the human male population, increasing with age up to fifty years, and between eighteen and seventy-five percent between fifty-five and eighty years of age. In the United States alone, for example, it has been estimated that there are up to ten million impotent males, with the majority suffering from problems of organic, rather than psychogenic origin.

Early reports of well-controlled clinical trials in man were few and the efficiency of orally administered agents was low. Although many different agents were shown to induce penile erection, they were effective only after direct injection into the penis, e.g. intraurethrally or intracavernosally (i.c.), and were not approved for erectile dysfunction. Other medical treatment was based upon the i.c. injection of vasoactive substances and satisfactory results were claimed with phenoxybenzamide, phentolamine, papverine and prostaglandin E₁, either alone or in combination; however, pain, priapism, and fibrosis of the penis was associated with the i.c. injection of some of these agents. Certain alternative methods of prostaglandin therapy in treating impotence and/or erectile dysfunction comprised the use of certain selective prostaglandin E₂ and/or E₄ receptor agonists, for example, those agonists disclosed in International Application Publication No. WO 9902164. Potassium channel openers (KCO) and vasoactive intestinal peptide (VIP) were also shown to be active when administered i.c., but cost and stability issues limited development of the latter. An alternative to the i.c. route was the use of glyceryl trinitrate (GTN) patches applied to the penis. This method was effective, but produced undesirable side-effects in both patient and partner. A variety of penile prostheses have been used to assist achievement of an erection. While the short term success rate is good, problems with sepsis and ischemia, especially in diabetic men, make this type of treatment a final option, rather than a first-line therapy.

More recently, the use of certain alternative drugs such as PDE₅ inhibitors, sildenafil for example, in the treatment of erectile dysfunction has proven very beneficial, and interest in the development of additional therapeutic agents remains high.

The present invention provides methods of treating impotence or erectile dysfunction which comprise administering to a patient in need of such treatment an impotence or erectile dysfunction treating amount of a prostaglandin receptor agonist shown and described in greater detail hereinbelow.

### SUMMARY OF THE INVENTION

The invention provides methods for treating impotence or erectile dysfunction which comprise administering to a patient in need of such treatment an impotence or erectile dysfunction treating amount of a compound selected from the group consisting of:
(i) a compound of Formula I 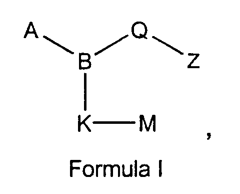
(ii) a compound of Formula II 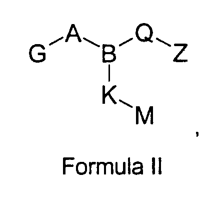
(iii) a compound of Formula III 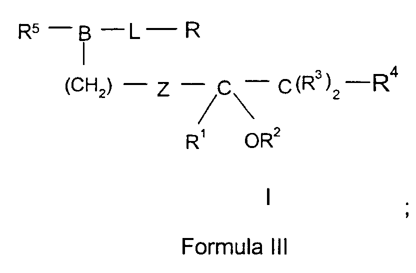 and
(iv) a compound of Formula IV 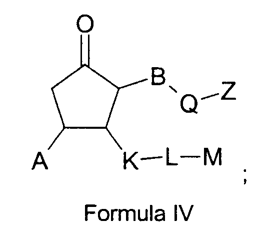 wherein the definitions of the various substituents in the Formula I, II, III, and IV compounds are as defined hereinbelow.

### DETAILED DESCRIPTION OF THE INVENTION

The invention provides methods for treating impotence or erectile dysfunction which comprise administering to a patient in need of such treatment an impotence or erectile dysfunction treating amount of a compound selected from the group consisting of:
(i) a compound of Formula I 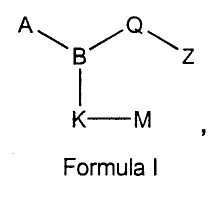 prodrugs thereof, and the pharmaceutically acceptable salts of the compounds and the prodrugs, wherein:
   B is N;
   A is (C₁-C₆)alkylsulfonyl, (C₃-C₇)cydoalkylsulfonyl, (C₃-C₇)cycloalkyl(C₁-C₆)alkylsulfonyl, said A moieties optionally mono-, di- or tri- substituted on carbon independently with hydroxy, (C₁-C₄)alkyl or halo;
   Q is
      -(C₂-C₆)alkylene-W-(C₁-C₃)alkylene-,
      -(C₃-C₈)alkylene-, said -(C₃-C₈)alkylene- optionally substituted with up to four substituents independently selected from fluoro or (C₁-C₄)alkyl,
      -X-(C₁-C₅)alkylene-,
      -(C₁-C₅)alkylene-X-,
      -(C₁-C₃)alkylene-X-(C₁-C₃)alkylene-,
      -(C₂-C₄)alkylene-W-X-(C₀-C₃)alkylene-,
      -(C₀-C₄)alkylene-X-W-(C₁-C₃)alkylene-,
      -(C₂-C₅)alkylene-W-X-W-(C₁-C₃)alkylene-, wherein the two occurrences of W are independent of each other,
      -(C₁-C₄)alkylene-ethenylene-(C₁-C₄)alkylene-,
      -(C₁-C₄)alkylene-ethenylene-(C₀-C₂)alkylene-X-(C₀-C₅)alkylene-,
      -(C₁-C₄)alkylene-ethenylene-(C₀-C₂)alkylene-X-W-(C₁-C₃)alkylene-,
      -(C₁-C₄)alkylene-ethynylene-(C₁-C₄)alkylene-, or
      -(C₁-C₄)alkylene-ethynylene-X-(C₀-C₃)alkylene-;
   W is oxy, thio, sulfino, sulfonyl, aminosulfonyl-, -mono-N-(C₁-C₄)alkyleneaminosulfonyl-, sulfonylamino, N-(C₁-C₄)alkylenesulfonylamino, carboxamido, N-(C₁-C₄)alkylenecarboxamido, carboxamidooxy, N-(C₁-C₄)alkylenecarboxamidooxy, carbamoyl, -mono-N-(C₁-C₄)alkylenecarbamoyl, carbamoyloxy, or -mono-N-(C₁-C₄)alkylenecarbamoyloxy, wherein said W alkyl groups are optionally substituted on carbon with one to three fluorines;
   X is a five- or six-membered aromatic ring optionally having one or two heteroatoms selected independently from oxygen, nitrogen, and sulfur; said ring optionally mono-, or di-substituted independently with halo, (C₁-C₃)alkyl, trifluoromethyl, trifluoromethyloxy, difluoromethyloxy, hydroxyl, (C₁-C₄)alkoxy, or carbamoyl;
   Z is carboxyl, (C₁-C₆)alkoxycarbonyl, tetrazolyl, 1,2,4-oxadiazolyl, 5-oxo-1,2,4-oxadiazolyl, (C₁-C₄)alkylsulfonylcarbamoyl or phenylsulfonylcarbamoyl;
   K is a bond, (C₁-C₈)alkylene, thio(C₁-C₄)alkylene or oxy(C₁-C₄)alkylene, said (C₁-C₈)alkylene optionally mono-unsaturated and wherein K is optionally mono-, di- or tri-substituted independently with fluoro, methyl or chloro;
   M is -Ar, -Ar¹-V-Ar², -Ar¹-S-Ar² or -Ar¹-O-Ar² wherein Ar, Ar¹ and Ar² are each independently a partially saturated, fully saturated or fully unsaturated five- to eight-membered ring optionally having one to four heteroatoms selected independently from oxygen, sulfur and nitrogen, or, a bicyclic ring consisting of two fused partially saturated, fully saturated or fully unsaturated five- or six-membered rings, taken independently, optionally having one to four heteroatoms selected independently from nitrogen, sulfur and oxygen;
   said Ar, Ar¹ and Ar² moieties optionally substituted, on one ring if the moiety is monocyclic, or one or both rings if the moiety is bicyclic, on carbon with up to three substituents independently selected from R¹, R² and R³ wherein R¹, R² and R³ are hydroxy, nitro, halo, (C₁-C₆)alkoxy, (C₁-C₄)alkoxy(C₁-C₄)alkyl, (C₁-C₄)alkoxycarbonyl, (C₁-C₇)alkyl, (C₃-C₇)cycloalkyl, (C₃-C₇)cycloalkyl(C₁-C₄)alkyl, (C₃-C₇)cydoalkyl(C₁-C₄)alkanoyl, formyl, (C₁-C₈)alkanoyl, (C₁-C₆)alkanoyl(C₁-C₆)alkyl, (C₁-C₄)alkanoylamino, (C₁-C₄)alkoxycarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl;
   R¹, R² and R³ are optionally mono-, di- or tri-substituted on carbon independently with halo or hydroxy; and
   V is a bond or (C₁-C₃)alkylene optionally mono- or di-substituted independently with hydroxy or fluoro;
(ii) a compound of Formula II 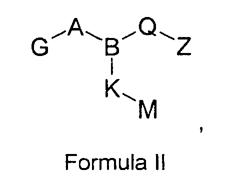 prodrugs thereof, and the pharmaceutically acceptable salts of the compounds and the prodrugs, wherein:
   A is SO₂ or CO;
   G is Ar, Ar¹-V-Ar², Ar-(C₁-C₆)alkylene, Ar-CONH-(C₁-C₆)alkylene, R¹R²-amino, oxy(C₁-C₆)alkylene, amino substituted with Ar, or amino substituted with Ar(C₁-C₄)alkylene and R¹¹, wherein R¹¹ is H or (C₁-C₈)alkyl, R¹ and R² may be taken separately and are independently selected from H and (C₁-C₈)alkyl, or R¹ and R² are taken together with the nitrogen atom of the amino group to form a five- or six-membered azacycloalkyl, said azacycloalkyl optionally containing an oxygen atom and optionally mono-, di- or tri-substituted independently with up to two oxo, hydroxy, (C₁-C₄)alkyl, fluoro or chloro;
   B is N or CH;
   Q is
      -(C₂-C₆)alkylene-W-(C₁-C₃)alkylene-, said alkylenes each optionally substituted with up to four substituents independently selected from fluoro or (C₁-C₄)alkyl,
      -(C₄-C₈)alkylene-, said alkylene optionally substituted with up to four substituents independently selected from fluoro or (C₁-C₄)alkyl,
      -X-(C₁-C₅)alkylene-, said alkylene optionally substituted with up to four substituents independently selected from fluoro or (C₁-C₄)alkyl,
      -(C₁-C₅)alkylene-X-, said alkylene optionally substituted with up to four substituents independently selected from fluoro or (C₁-C₄)alkyl,
      -(C₁-C₃)alkylene-X-(C₁-C₃)alkylene-, said alkylenes each optionally substituted with up to four substituents independently selected from fluoro or (C₁-C₄)alkyl,
      -(C₂-C₄)alkylene-W-X-(C₀-C₃)alkylene-, said alkylenes each optionally substituted with up to four substituents each independently selected from fluoro or
      (C₁-C₄)alkyl,
      -(C₀-C₄)alkylene-X-W-(C₁-C₃)alkylene-, said alkylenes each optionally substituted with up to four substituents each independently selected from fluoro or
      (C₁-C₄)alkyl,
      -(C₂-C₅)alkylene-W-X-W-(C₁-C₃)alkylene-, wherein the two occurrences of W are independent of each other, said alkylenes each optionally substituted with up to four substituents each independently selected from fluoro or (C₁-C₄)alkyl,
      -(C₁-C₄)alkylene-ethenylene-(C₁-C₄)alkylene-, said alkylenes and said ethenylene each optionally substituted with up to four substituents each independently selected from fluoro or (C₁-C₄)alkyl,
      -(C₁-C₄)alkylene-ethenylene-(C₀-C₂)alkylene-X-(C₀-C₅)alkylene-, said alkylenes and said ethenylene each optionally substituted with up to four substituents each independently selected from fluoro or (C₁-C₄)alkyl,
      -(C₁-C₄)alkylene-ethenylene-(C₀-C₂)alkylene-X-W-(C₁-C₃)alkylene-, said alkylenes and said ethenylene each optionally substituted with up to four substituents each independently selected from fluoro or (C₁-C₄)alkyl,
      -(C₁-C₄)alkylene-ethynylene-(C₁-C₄)alkylene-, said alkylenes and said ethynylene each optionally substituted with up to four substituents each independently selected from fluoro or (C₁-C₄)alkyl, or
      -(C₁-C₄)alkylene-ethynylene-X-(C₀-C₃)alkylene-, said alkylenes and said ethynylene each optionally substituted with up to four substituents each independently selected from fluoro or (C₁-C₄)alkyl;
   Z is carboxyl, (C₁-C₆)alkoxycarbonyl, tetrazolyl, 1,2,4-oxadiazolyl, 5-oxo-1,2,4-oxadiazolyl, 5-oxo-1,2,4-thiadiazolyl, (C₁-C₄)alkylsulfonylcarbamoyl or phenylsulfonylcarbamoyl;
   K is a bond, (C₁-C₉)alkylene, thio(C₁-C₄)alkylene, (C₁-C₄)alkylenethio(C₁-C₄)alkylene, (C₁-C₄)alkyleneoxy(C₁-C₄)alkylene or oxy(C₁-C₄)alkylene, said (C₁-C₉)alkylene optionally mono-unsaturated and wherein, when K is not a bond, K is optionally mono-, di- or tri-substituted independently with chloro, fluoro, hydroxy or methyl;
   M is -Ar³, -Ar⁴-V¹-Ar⁵, -Ar⁴-S-Ar⁵, -Ar⁴-SO-Ar⁵, -Ar⁴-SO₂-Ar⁵ or -Ar⁴-O-Ar⁵;
   Ar is a partially saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five- or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, taken independently, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur; or Ar is a fully saturated five-to seven-membered ring having one or two heteroatoms selected independently from oxygen, sulfur and nitrogen;
   Ar¹ and Ar² are each independently a partially saturated, fully saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five- or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur;
   said Ar, Ar¹ and Ar² moieties are optionally substituted on carbon or nitrogen, on one ring if the moiety is monocyclic, on one or both rings if the moiety is bicyclic, or on one, two or three rings if the moiety is tricyclic, with up to three substituents per moiety independently selected from R³, R⁴ and R⁵ wherein R³, R⁴ and R⁵ are independently hydroxy, nitro, halo, carboxy, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, (C₁-C₄)alkanoylamino, (C₁-C₄)alkoxycarbonylamino, hydroxysulfonyl, aminocarbonylamino or mono-N-, di-N,N-, di-N,N'- or tri-N,N,N'-(C₁-C₄)alkyl substituted aminocarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl;
   Ar³, Ar⁴ and Ar⁵ are each independently a partially saturated, fully saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five- or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur;
   said Ar³, Ar⁴ and Ar⁵ moieties are optionally substituted on carbon or nitrogen, on one ring if the moiety is monocyclic, on one or both rings if the moiety is bicyclic, or on one, two or three rings if the moiety is tricyclic, with up to three substituents per moiety independently selected from R³¹, R⁴¹ and R⁵¹ wherein R³¹, R⁴¹ and R⁵¹ are independently hydroxy, nitro, halo, carboxy, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, (C₁-C₄)alkanoylamino, (C₁-C₄)alkoxycarbonylamino, hydroxysulfonyl, aminocarbonylamino or mono-N-, di-N,N-, di-N,N'- or tri-N,N,N'-(C₁-C₄)alkyl substituted aminocarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl;
   W is oxy, thio, sulfino, sulfonyl, aminosulfonyl-, -mono-N-(C₁-C₄)alkyleneaminosulfonyl-, sulfonylamino, N-(C₁-C₄)alkylenesulfonylamino, carboxamido, N-(C₁-C₄)alkylenecarboxamido, carboxamidooxy, N-(C₁-₄)alkylenecarboxamidooxy, carbamoyl, -mono-N-(C₁-C₄)alkylenecarbamoyl, carbamoyloxy, or -mono-N-(C₁-C₄)alkylenecarbamoyloxy, wherein said W alkyl groups are optionally substituted on carbon with one to three fluorines;
   X is a five- or six-membered aromatic ring optionally having one or two heteroatoms selected independently from oxygen, nitrogen, and sulfur; said ring optionally mono-, di- or tri-substituted independently with halo, (C₁-C₃)alkyl, trifluoromethyl, trifluoromethyloxy, difluoromethyloxy, hydroxyl, (C₁-C₄)alkoxy, or carbamoyl;
   R¹, R², R³, R⁴ R⁵, R¹¹, R³¹, R⁴¹ and R⁵¹, when containing an alkyl, alkylene, alkenylene or alkynylene moiety, are optionally mono-, di- or tri-substituted on carbon independently with halo or hydroxy; and
   V and V¹ are each independently a bond, thio(C₁-C₄)alkylene, (C₁-C₄)alkylenethio, (C₁-C₄)alkyleneoxy, oxy(C₁-C₄)alkylene or (C₁-C₃)alkylene optionally mono- or di-substituted independently with hydroxy or fluoro;
(iii) a compound of Formula III 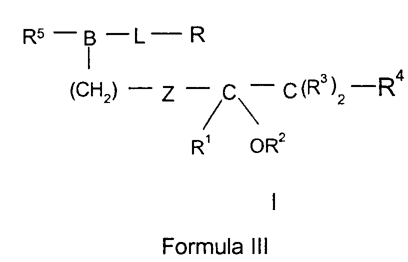 prodrugs thereof, and the pharmaceutically acceptable salts of the compounds and the prodrugs, wherein:
   B is N or C(Q¹), where Q¹ is H or (C₁-C₃)alkyl;
   L is n-propylenyl-X- or CH₂-metaphenylene-CH₂, wherein X is furanyl, thienyl, thiazolyl or tetrahydrofuranyl, said CH₂-metaphenylene-CH₂ or X being optionally mono-, di- or tri-substituted on aromatic carbon independently with one to three chloro, fluoro, methoxy, difluoromethoxy, trifluoromethoxy, trifluoromethyl or methyl;
   R is carboxyl, (C₁-C₆)alkoxycarbonyl, tetrazolyl, 5-oxo-1,2,4-thiadiazolyl; 5-oxo-1,2,4-oxadiazolyl, (C₁-C₄)alkylsulfonylcarbamoyl or phenylsulfonylcarbamoyl;
   R¹ is H, methyl, ethyl or propyl;
   R² is H or (C₂ - C₅) alkanoyl;
   R³ is independently H, fluoro or methyl;
   R⁴ is H, (C₁ - C₇) alkyl, or R⁴ and R¹ are taken together to form a 5-9 membered carbocyclic ring, said alkyl being optionally monounsaturated and optionally mono-, di- or tri-substituted independently with one to three fluoro, chloro, methoxy, difluoromethoxy, trifluoromethoxy, trifluoromethyl or methyl;
   R⁵ is (C₁-C₆)alkylsulfonyl, (C₃-C₇)cycloalkylsulfonyl, (C₃-C₇)cydoalkyl(C₁-C₆)alkylsulfonyl, (C₁-C₆)alkylcarbonyl, (C₃-C₇)cydoalkylcarbonyl, (C₃-C₇)cydoalkyl(C₁-C₆)alkylcarbonyl, G-sulfonyl or G-carbonyl, said (C₁-C₆)alkylsulfonyl, (C₃-C₇)cycloalkylsulfonyl, (C₃-C₇)cycloalkyl(C₁-C₆)alkylsulfonyl, (C₁-C₆)alkylcarbonyl, (C₃-C₇)cycloalkylcarbonyl, (C₃-C₇)cycloalkyl(C₁-C₆)alkylcarbonyl optionally mono-, di- or tri- substituted on carbon independently with hydroxy, fluoro, chloro, methoxy, difluoromethoxy, trifluoromethoxy, trifluoromethyl or methyl;
   Z is methylene, ethylene, propylene or ethenylene;
   G is Ar, Ar¹-V-Ar², Ar-(C₁-C₆)alkylene, Ar-CONH-(C₁-C₆)alkylene, R¹²R¹³-amino, oxy(C₁-C₆)alkylene, amino substituted with Ar, or amino substituted with Ar(C₁-C₄)alkylene and R¹¹, wherein R¹¹ is H or (C₁-C₈)alkyl, R¹² and R¹³ may be taken separately and are independently selected from H and (C₁-C₈)alkyl, or R¹² and R¹³ are taken together with the nitrogen atom to which they are attached to form a five- or six-membered azacycloalkyl, said azacycloalkyl optionally containing an oxygen atom and optionally substituted with up to two oxo, hydroxy, (C₁-C₄)alkyl, fluoro or chloro;
   Ar is a partially saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five- or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur; or Ar is a fully saturated five to seven-membered ring having one or two heteroatoms selected independently from oxygen, sulfur and nitrogen;
   Ar¹ and Ar² are each independently a partially saturated, fully saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five- or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, taken independently, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur;
   said Ar, Ar¹ and Ar² moieties are optionally substituted on carbon or nitrogen, on one ring if the moiety is monocyclic, on one or both rings if the moiety is bicyclic, or on one, two or three rings if the moiety is tricyclic, with up to three substituents per moiety, independently selected from R¹⁴, R¹⁵ and R¹⁶ wherein R¹⁴, R¹⁵ and R¹⁶ are independently hydroxy, nitro, halo, carboxy, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, (C₁-C₄)alkanoylamino, (C₁-C₄)alkoxycarbonylamino, hydroxysulfonyl, aminocarbonylamino or mono-N-, di-N,N-, di-N,N'- or tri-N,N,N'-(C₁-C₄)alkyl substituted aminocarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl; and
   V is a bond, thio(C₁-C₄)alkylene, (C₁-C₄)alkylenethio, (C₁-C₄)alkyleneoxy, oxy(C₁-C₄)alkylene or (C₁-C₃)alkylene optionally mono- or di-substituted, when V is not a bond, independently with hydroxy or fluoro; and
(iv) a compound of Formula IV 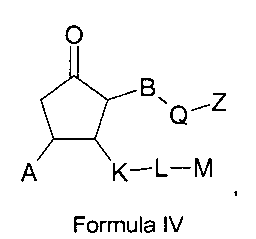 prodrugs thereof, and the pharmaceutically acceptable salts of the compounds and the prodrugs wherein:
   A is hydrogen or hydroxy;
   B is propylene, propenylene or propynylene;
   Q is propylene, -CH₂OCH₂-, thiazolyl, pyridyl, phenyl or thienyl;
   Z is carboxyl, (C₁-C₆)alkoxycarbonyl, tetrazolyl, 1,2,4-oxadiazolyl or 5-oxo-1,2,4-oxadiazolyl;
   K is ethylene or ethenylene;
   L is a bond or -CO-;
   M is -Ar, -Ar¹-V-Ar², -Ar¹-S-Ar² or -Ar¹-O-Ar² wherein
   Ar and Ar¹ are either
      (1) each independently a fully unsaturated five- to eight-membered ring optionally having one to four heteroatoms selected independently from oxygen, sulfur and nitrogen, or a bicyclic ring consisting of two fused partially saturated, fully saturated or fully unsaturated five- and/or six-membered rings, taken independently, optionally having one to four heteroatoms selected independently from nitrogen, sulfur and oxygen, or a tricyclic ring consisting of three fused partially saturated, fully saturated or fully unsaturated five- and/or six-membered rings, taken independently, optionally having one to four heteroatoms selected independently from nitrogen, sulfur and oxygen, any of said partially saturated or fully saturated rings optionally having one or more oxo groups substituted on carbon, or
      (2) each independently a fully saturated five to eight membered ring;
   Ar² is a partially saturated, fully saturated or fully unsaturated five- to eight-membered ring optionally having one to four heteroatoms selected independently from oxygen, sulfur and nitrogen, or a bicyclic ring consisting of two fused partially saturated, fully saturated or fully unsaturated five- and/or six-membered rings, taken independently, optionally having one to four heteroatoms selected independently from nitrogen, sulfur and oxygen, or a tricyclic ring consisting of three fused partially saturated, fully saturated or fully unsaturated five- and/or six-membered rings, taken independently, optionally having one to four heteroatoms selected independently from nitrogen, sulfur and oxygen, any of said partially saturated or fully saturated rings optionally having one or more oxo groups substituted on carbon;
   said Ar and Ar¹ moieties, when a fully unsaturated five- to eight-membered ring, a bicyclic ring or a tricyclic ring, and said Ar² moieties are each independently optionally substituted on carbon, on one ring if the moiety is monocyclic, on one or both rings if the moiety is bicyclic, or on one, two or three rings if the moiety is tricyclic, with up to three substituents selected from R¹, R² and R³ wherein R¹, R² and R³ are independently hydroxy, nitro, halo, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, aminocarbonylamino or mono-N-, di-N,N-, di-N,N'- or tri-N,N,N'-(C₁-C₄)alkyl substituted aminocarbonylamino, (C₁-C₄)alkanoylamino, (C₁-C₄)alkoxycarbonylamino, sulfonamido, hydroxysulfonyl, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl;
   R¹, R² and R³, when containing an alkyl, alkenyl, alkylene or alkenylene moiety, are optionally straight or branched and are optionally mono-, di- or tri-substituted on carbon independently with halo or hydroxy; and
   V is a bond, -CO- or (C₁-C₃)alkylene optionally mono- or di-substituted independently with hydroxy or fluoro.

A preferred subgroup of Formula I compounds comprises those compounds selected from:
7-[(2'-hydroxymethyl-biphenyl-4-ylmethyl)-methanesulfonyl-amino]-heptanoic acid;
7-{[4-(3-hydroxymethyl-thiophen-2-yl)-benzyl]-methanesulfonyl-amino}-heptanoic acid;
7-[(2'-chloro-biphenyl-4-ylmethyl)-methanesulfonyl-amino]-heptanoic acid;
7-{[4-(1-hydroxy-hexyl)-benzyl]-methanesulfonyl-amino}-heptanoic acid;
7-[(4-butyl-benzyl)-methanesulfonyl-amino]-heptanoic acid;
7-{[5-(1-hydroxy-hexyl)-thiophen-2-ylmethyl]-methanesulfonyl-amino}-heptanoic acid;
(3-{[(4-butyl-benzyl)-methanesufonyl-amino]-methyl}-phenyl)-acetic acid;
7-{[3-(3-chloro-phenyl)-propyl]-methanesulfonyl-amino}-heptanoic acid;
7-{[3-(3,5-dichloro-phenyl)-propyl]-methanesufonyl-amino}-heptanoic acid;
5-(3-{[3-(3-chloro-phenyl)-propyl]-methanesulfonyl-amino}-propyl)-thiophene-2-carboxylic acid;
7-{[2-(3,5-dichloro-phenoxy)-ethyl]-methanesulfonyl-amino}-heptanoic acid;
5-(3-{[2-(3,5-dichloro-phenoxy)-ethyl]-methanesulfonyl-amino}-propyl)-thiophene-2-carboxylic acid;
N-[2-(3,5-dichloro-phenoxy)-ethyl]-N-[6-(1H-tetrazol-5-yl)-hexyl]-methanesulfonamide;
trans-(4-{[3-(3,5-dichloro-phenyl)-allyl]-methanesulfonyl-amino}-butoxy)-acetic acid;
*trans*-N-[3-(3,5-dichloro-phenyl)-allyl]-N-[6-(1H-tetrazol-5-yl)-hexyl]-methanesulfonamide;
trans-5-(3-{[3-(3,5-dichloro-phenyl)-allyl]-methanesulfonyl-amino}-propyl)-thiophene-2-carboxylic acid; and
trans-[3-({[3-(3,5-dichloro-phenyl)-allyl]-methanesulfonyl-amino}-methyl)-phenyl]-acetic acid; the prodrugs thereof, and the pharmaceutically acceptable salts of the compounds, and the prodrugs.

A preferred subgroup of Formula II compounds comprises those compounds selected from:
(3-(((pyridine-3-sulfonyl)-(4-pyrimidin-5-yl-benzyl)-amino)-methyl)-phenyl)-acetic acid;
(3-(((5-phenyl-furan-2-ylmethyl)-(pyridine-3-sulfonyl)-amino)-methyl)-phenyl)-acetic acid;
(3-(((pyridine-3-sulfonyl)-(4-pyrimidin-2-yl-benzyl)-amino)-methyl)-phenyl)-acetic acid;
(3-(((pyridine-3-sulfonyl)-(4-thiazol-2-yl-benzyl)-amino)-methyl)-phenyl)-acetic acid;
(3-(((4-pyrazin-2-yl-benzyl)-(pyridine-3-sulfonyl)-amino)-methyl)-phenyl)-acetic acid;
(3-(((4-cyclohexyl-benzyl)-(pyridine-3-sulfonyl)-amino)-methyl)-phenoxy)-acetic acid;
(3-(((pyridine-3-sulfonyl)-(4-pyridin-2-yl-benzyl)-amino)-methyl)-phenoxy)-acetic acid;
(3-(((pyridine-3-sulfonyl)-(4-pyridin-3-yl-benzyl)-amino)-methyl)-phenoxy)-acetic acid;
(3-(((pyridine-3-sulfonyl)-(4-pyridin-4-yl)-benzyl)-amino)-methyl)-phenoxy)-acetic acid;
(3-(((pyridine-3-sulfonyl)-(4-thiazol-2-yl-benzyl)-amino)-methyl)-phenoxy)-acetic acid;
(3-(((2,3-dihydro-benzo[1,4]dioxin-6-ylmethyl)-(pyridine-3-sulfonyl)-amino)-methyl)-phenyl)-acetic acid;
(3-(((benzofuran-2-ylmethyl-(pyridine-3-sulfonyl)-amino)-methyl)-phenyl)-acetic acid;
(3-(((4-butyl-benzyl)-(pyridine-3-sulfonyl)-amino)-methyl)-phenyl)-acetic acid;
(3-(((benzenesulfonyl-(4-butyl-benzyl)-amino)-methyl)-phenyl)-acetic acid;
(3-(((4-butyl-benzyl)-(1-methyl-1H-imidazole-4-sulfonyl)-amino)-methyl)-phenyl)-acetic acid;
(3-(((4-dimethylamino-benzyl)-(pyridine-3-sulfonyl)-amino)-methyl)-phenyl)-acetic acid;
(3-(((4-dimethylamino-benzyl)-(pyridine-3-sulfonyl)-amino)-methyl)-phenoxy)-acetic acid;
(3-(((4-tert-butyl-benzyl)-(pyridine-3-sulfonyl)-amino)-methyl)-phenoxy)-acetic acid;
*trans*-(3-(((3-(3,5-dichloro-phenyl)-allyl)-(pyridine-3-sulfonyl)-amino)-methyl)-phenyl)-acetic acid; and
(3-(((2-(3,5-dichloro-phenoxy)-ethyl)-(pyridine-3-sulfonyl)-amino)-methyl)-phenoxy)-acetic acid; the prodrugs thereof, and the pharmaceutically acceptable salts of the compounds, and the prodrugs.

A preferred subgroup of Formula III compounds comprises those compounds wherein:
B is N; R is carboxyl, (C₁-C₆)alkoxycarbonyl or tetrazolyl; Z is ethylenyl; R¹ and R² are each H; and L is CH₂-metaphenylene-CH₂ or n-propylene-X-; the prodrugs thereof, and the pharmaceutically acceptable salts of the compounds, and the prodrugs.

A further preferred subgroup of Formula III compounds comprises those compounds wherein:
R⁵ is selected from (C₁-C₆)alkycarbonyl, optionally mono-, di-, or tri-substituted with hydroxy or fluoro; (C₁-C₃)alkysulfonyl or (C₃-C₇)cycloalkysulfonyl; and G-sulfonyl, wherein G is phenyl, imidazolyl, pyridyl, pyrazolyl, or pyrimidyl optionally mono-, di-, or tri-substituted on carbon or nitrogen with chloro, fluoro, methoxy, difluoromethoxy, trifluoromethoxy, trifluoromethyl or methyl; the prodrugs thereof, and the pharmaceutically acceptable salts of the compounds, and the prodrugs.

A preferred subgroup of Formula IV compounds comprises those compounds selected from:
*trans*-7-(2-(2-(3,5-bis-trifluoromethyl-phenyl)-vinyl)-5-oxo-cyclopentyl)-heptanoic acid;
*trans*-7-(2-(2-(4-chloro-3-trifluoromethyl-phenyl)-vinyl)-5-oxo-cyclcopentyl)-heptancic acid;
*trans*-7-(2-(2--(3,5-dichlorophenyl)-vinyl-5-oxo-cyclopentyl)-heptanoic acid;
*trans*-7-(2-(2-(3-chlorophenyl-vinyl)-5-oxo-cyclopentyl)-heptanoic acid;
*trans*-7-(2-oxo-5-(2-(3-trifluoromethyl-phenyl)-vinyl)-cydopentyl)-heptanoic acid;
*trans*-7-(2-(2-(4-fluoro-phenyl)-vinyl)-5-oxo-cyclopentyl)-heptanoic acid;
ethyl *trans*-7-(2-(2-(3,5-bis-trifluoromethyl-phenyl)-vinyl)-5-oxocyclopentyl)-heptanoate;
ethyl *trans*-7-(2-(2-(4-chloro-3-trifluoromethyl-phenyl)-vinyl)-5-oxo-cyclopentyl)-heptanoate;
ethyl *trans*-7-(2-(2-(3,5-dichlorophenyl)-vinyl)-5-oxo-cyclopentyl)-heptanoate;
ethyl *trans*-7-(2-(2-(3-chlorophenyl)-vinyl)-5-oxo-cyclopentyl)-heptanoate;
ethyl *trans*-7-(2-oxo-5-(2-(3-trifluoromethyl-phenyl)-vinyl)-cyclopentyl)-heptanoate;
ethyl *trans*-7-(2-(2-(4-fluoro-phenyl)-vinyl)-5-oxo-cyclopentyl)-heptanoate;
*trans*-3-(2-(3,5-bis-trifluoromethyl-phenyl)-vinyl)--2-(6-(2H-tetrazol-5-yl)-hexyl)-cyclopentanone;
*trans*-3-(2-(4-chloro-3-trifluoromethylphenyl)-vinyl)-2-(6-(2H-tetrazol-5-yl)-hexyl)-cyclopentanone;
*trans*-3-(2-(3,5-dichloro-phenyl)-vinyl)-2-(6-(2H-tetrazol-5-yl)-hexyl)-cyclopentanone;
*trans*-3-(2-(3-chloro-phenyl)-vinyl)-2-(6-(2H-tetrazol-5-yl)-hexyl)-cyclopentanone;
*trans*-3-(2-(3-trifluoromethyl-phenyl)-vinyl)-2-(6-(2H-tetrazol-5-yl)-hexyl)-cyclopentanone; and
*trans*-3-(2-(4-fluoro-phenyl)-vinyl)-2-(6-(2H-tetrazol-5-yl)-hexyl)-cyclopentanone; the prodrugs thereof, and the pharmaceutically acceptable salts of the compounds, and the prodrugs.

The compounds of Formula I, the prodrugs thereof, and the pharmaceutically acceptable salts of the compounds and the prodrugs, may be prepared according to the synthetic methodologies described in International Application Publication No. WO 98/28264.

The compounds of Formula II, the prodrugs thereof, and the pharmaceutically acceptable salts of the compounds and the prodrugs, may be prepared according to the synthetic methodologies described in International Application Publication No. WO 99/19300.

The compounds of Formula III, the prodrugs thereof, and the pharmaceutically acceptable salts of the compounds and the prodrugs, may be prepared according to the synthetic methodologies described in European Patent Application Publication No. EP 0 911 321.

The compounds of Formula IV, the prodrugs thereof, and the pharmaceutically acceptable salts of the compounds and the prodrugs, may be prepared according to the synthetic methodologies described in International Application Publication No. WO 98/58911.

The dosage of the compounds to be administered in accordance with the methods of the invention will generally be dependent upon a number of factors including the health of the subject being treated, the extent of treatment desired, the nature and kind of concurrent therapy, if any, and the frequency of treatment desired. In general, representative dosages range from about 0.1 mg/kg/day to about 30.0 mg/kg/day. Generally, preferable dosages range from about 1.0 mg/kg/day to about 10.0 mg/kg/day. However, some variability in the general dosage range may be required depending upon the age and weight of the patient, the intended route of administration, and the like.

According to the methods of the invention, the compounds are administered preferably in the form of a pharmaceutical composition comprising a pharmaceutically acceptable carrier, vehicle or diluent. Accordingly, a compound of the invention may be administered in any conventional oral, parenteral, or transdermal dosage form.

Suitable pharmaceutically-acceptable carriers include inert solid fillers or diluents and sterile aqueous or organic solutions. According to the methods of the invention, a compound of the invention will be present in such pharmaceutical compositions in amounts sufficient to provide the desired dosage amount in the ranges described hereinabove. Thus, for oral administration, the compounds can be combined with a suitable solid or liquid carrier, vehicle or diluent to form capsules, tablets, powders, syrups, solutions, suspensions and the like. The pharmaceutical compositions may, if desired, contain additional components such as flavorants, sweeteners, excipients and the like.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

The pharmaceutical compositions described hereinabove may also be administered parenterally. For parenteral administration the pharmaceutical compositions can be combined with sterile aqueous or organic media to form injectable solutions or suspensions. Solutions or suspensions of these pharmaceutical compositions can be prepared in water suitably mixed with a surfactant such as hydroxypropylcellulose. Dispersions can also be prepared in sesame or peanut oil, ethanol, water, polyol (e.g., glycerol, propylene glycol and liquid polyethylene glycol), suitable mixtures thereof, vegetable oils, N-methyl glucamine, polyvinylpyrrolidone and mixtures thereof in oils as well as aqueous solutions of water-soluble pharmaceutically acceptable salts of the compounds. Under ordinary conditions of storage and use, these preparations contain a preservative to prevent the growth of microorganisms. The injectable solutions prepared in this manner can then be administered intravenously, intraperitoneally, subcutaneously, or intramuscularly, with intramuscular administration being the preferred parenteral route in humans. Solutions prepared for intravenous administration are preferably rendered isotonic prior to usage.

The pharmaceutical forms suitable for injectable use include sterile aqueous solutions or dispersions and sterile powders for the preparation of sterile injectable solutions or dispersions. In all cases, the form must be sterile and must be fluid to the extent that easy syringability exists. It must be stable under the conditions of manufacture and storage and preserved against contamination by microorganisms such as bacteria and fungi.

The pharmaceutical compositions may also be administered transdermally. Suitable formulations for transdermal application include an impotence or erectile dysfunction treating amount of a compound of the invention, or pharmaceutical composition thereof, with a suitable transdermal carrier. Preferred transdermal carriers include absorbable pharmacologically acceptable solvents to promote and assist passage through the skin of the subject being treated. If the composition is to be administered transdermally, various creams, ointments, gels, and slow release systems such as bandages may be employed. Characteristically, transdermal devices comprising the form of a bandage have a backing member, a reservoir containing the compound, optionally with carriers, optionally a rate-controlling barrier to deliver the compound to the skin of the subject being treated at a controlled and predetermined rate over a prolonged period of time and means to secure the device to the skin of the subject being treated. Alternatively, the inner surface of a condom may be lined with a suitable formulation comprising the medicament and the transdermal carrier. If the medicament is to be administered transdermally as a cream, ointment, or gel, the formulation may optionally comprise preservatives such as benzalkonium chloride, chlorhexidine, thiomersal, parabenzoic acid, and other compounds with antimicrobial properties.

Methods of preparing the various pharmaceutical compositions with a desired amount of an active ingredient are known, or will be apparent in light of this disclosure, to one of ordinary skill in the art. See, for example, Remington's Pharmaceutical Sciences, Mack Publishing Company, Easton, PA, 18th Edition (1990).

### EXPERIMENTAL

The utility of the compounds of the instant invention in treating impotence or erectile dysfunction may be demonstrated according to the following protocol.

### Rabbit Corpus Cavernosum in vitro Preparation

Male New Zealand white rabbits (Harlan Sprague-Dawley, Inc.; Indianapolis, IN) (2.0 - 3.0 kg) are killed by cervical dislocation. The abdominal cavity is opened and the penis excised, starting at the base of the organ at the pelvic bones. The corpus cavernosum tissue is seen as a pair of spongy columns of tissue which lie along the ventral side of the penis and surrounded by a ring of dense connective tissue. A single incision along the length of the connective tissue using a scalpel is made to expose the corpus cavernosum which is then excised using a pair of small bow spring scissors. Two tissues strips approximately 10mm x 2mm x 2mm in size are obtained from each rabbit.

Tissues strips are mounted longitudinally in 10ml silanised organ chambers with braided silk sutures (6/0 gauge) at an initial resting tension of 2g in Krebs bicarbonate buffer maintained at 37°C and gassed with 95%02/5%CO₂. The upper ligature of each tissue strip is attached to a 10g capacity force-displacement transducer and changes in isometric force are measured and recorded.

Tissues are allowed to equilibrate for 1.5 hours. After equilibration, the tissues are depolarised using 120mM bath concentration of KCI for 5 minutes and are then allowed to return to baseline tension by washing with Krebs-solution modified to contain 1µM atropine and 5µM guanethidine (to ensure that no adrenergic or cholinergic component is present) for 30 minutes.

### Sodium Nitronrusside-lnduced Relaxation of Rabbit Corpus Cavernosum

Each tissue is contracted using 10µM bath concentration of phenylephrine. When the contractile response reaches a stable plateau (15 minutes), sodium nitroprusside (SNP) is cumulatively added to the organ chamber at half log units to produce concentrations from 0.01 - 100µM. The relaxation responses are measured 2 minutes after the addition of each concentration of SNP; maximum relaxation is achieved by this time. After completion of the pre-treatment relaxation response curve, all tissues are washed for 15 minutes, allowing the tissues to return to the baseline tension. Tissues then receive either the test compound or DMSO vehicle (time matched control). Tissues are re-contracted with phenylephrine 15 minutes after the addition of compound or vehicle and a SNP relaxation response curve determined as described above.

In each experiment, compounds are evaluated at 3 sequentially increasing concentrations per tissue. Curve 1 is the pre-treatment curve and is performed in the absence of compound or vehicle. Curves 2, 3, and 4 are carried out either in the presence of increasing concentrations of compound (treated tissues) or vehicle (time matched controls).

### Analysis of Data for SNP Relaxation Experiments

For each SNP concentration relaxation-response curve, the relaxation responses induced by SNP are expressed as a percentage of the phenylephrine induced contraction. These values are then plotted against log SNP concentration and sigmoidal curves are fitted. For the purpose of curve fitting the minimum relaxation response is constrained to 0 % and the maximum relaxation response is allowed to free fit. The concentration of SNP required to produce 50 % relaxation of the phenylephrine contraction (EC_{50 PE} ) is determined.

The EC_{50 PE} values are expressed as pD₂ (negative log EC_{50 PE}), which are then plotted against the log compound concentration The relative functional potency of the test compound is determined by interpolating the concentration of compound required to increase the pD₂ to 5.7. The pD₂ value of 5.7 is an arbitrary value chosen to allow the rank order of test compound potencies to be determined; it is equivalent to a reduction of EC_{50 PE} to 2µM.

### Electrical Field-Stimulated Relaxation of Rabbit Corpus Cavernosum

Rabbit corpus cavernosum strips are prepared as described hereinabove, but are mounted in 5ml silanised organ chambers instead of the 10ml silanised organ chambers. The tissue strips are mounted between two platinum electrodes placed at the top and bottom of the organ chamber approximately 4 cm apart.

Each tissue is pre-treated with vehicle (DMSO) for 15 minutes before being pre-contracted using 10µM organ chamber concentration of phenylephrine. After the phenylephrine contractile responses reach a stable plateau (15 minutes), the tissues undergo a pre-treatment electrical field stimulated (EFS) induced relaxation curve. This is performed between 40 - 45 volts using sequential frequencies of 2, 4, 8 and 16 Hz delivered as 10 second trains of 2 millisecond pulse width. The tissues are allowed to return to base line pre-contractile tension between each frequency (2 minutes) and the size of the relaxation response recorded. To reduce variability, tissues which do not induce a relaxation response between 30 and 90% of the phenylephrine contraction at 8Hz during the pre-treatment EFS are discarded.

After completion of the pre-treatment EFS response curve, all tissues are washed for 15 minutes, allowing the tissues to return to the baseline tension. Tissues then receive either the test compound or DMSO vehicle (time matched control). Tissues are re-contracted with phenylephrine 15 minutes after the addition of compound or vehicle and an EFS-induced relaxation response curve is determined as described hereinabove.

In each experiment, the test compound is evaluated at 3 sequentially increasing concentrations per tissue. Curve 1 is the pre-treatment curve and is performed in the presence of vehicle. Curves 2, 3, and 4 are carried out either in the presence of increasing concentrations of compound (treated tissues) or vehicle.

### Analysis of Data for EFS Experiments

The magnitude of the relaxation response to EFS is determined for each frequency and expressed as a % of the phenylephrine contraction. EFS-induced relaxation responses in the presence of the test compound are compared with the pre-treatment response. Time-matched controls (vehicle only) are monitored to show that no non-test compound related changes in EFS responses are occurring during the course of the experiments.

### Materials

Krebs bicarbonate buffer: NaCl 118.0mM; NaHCO₃ 25.0mM; KCl 4.7mM; KH₂PO₄ 1.2mM; MgSO₄.7H₂0 1.2mM; Glucose 11mM; CaCl₂ 1.5mM. Krebs solution is aerated with (95%02/5%CO₂) throughout the day.

KCI: 3.6M dissolved in distilled water.

Phenylephrine hydrochloride (Sigma, P-6126): 10mM dissolved in distilled water.

Atropine sulphate salt (Sigma, A-0257): 1mM dissolved in distilled water.

Guanethidine monosulphate (Sigma, G-8520); 5mM dissolved in distilled water.

Sodium nitroprusside dihydrate (Sigma, S-0501): 100mM stock is dissolved in distilled water which is then serially diluted to 10mM, 1mM, 100µM and 10µM stocks. Solutions are stored on ice.

Prostaglandin receptor agonists: stock solutions are prepared in 100% DMSO at 1000-fold the final bath concentration (10µl added to a 10ml of Krebs buffer).

## Claims

1. The use of a compound for the manufacture of a medicament for treating impotence or erectile dysfunction wherein the compound is selected from the group consisting of:
(i) a compound of Formula I 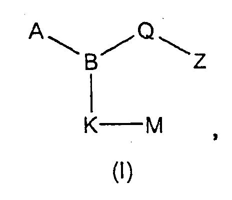 prodrugs thereof, and the pharmaceutically acceptable salts of said compounds and said prodrugs, wherein
B is N;
A is (C₁-C₆)alkylsulfonyl, (C₃-C₇)cycloalkylsulfonyl, (C₃-C₇)cycloalkyl(C₁-C₆)alkylsulfonyl, said A moieties optionally mono-, di- or tri- substituted on carbon independently with hydroxy, (C₁-C₄)alkyl or halo;
Q is
-(C₂-C₆)alkylene-W-(C₁-C₃)alkylene-,
-(C₃-C₈)alkylene-, said -(C₃-C₈)alkylene- optionally substituted with up to four substituents independently selected from fluoro or (C₁-C₄)alkyl,
-X-(C₁-C₅)alkylene-,
-(C₁-C₅)alkylene-X-,
-(C₁-C₃)alkylene-X-(C₁-C₃)alkylene-,
-(C₂-C₄)alkylene-W-X-(C₀-C₃)alkylene-,
-(C₀-C₄)alkylene-X-W-(C₁-C₃)alkylene-,
-(C₂-C₅)alkylene-W-X-W-(C₁-C₃)alkylene-, wherein the two occurrences of W are independent of each other,
-(C₁-C₄)alkylene-ethenylene-(C₁-C₄)alkylene-,
-(C₁-C₄)alkylene-ethenylene-(C₀-C₂)alkylene-X-(C₀-C₅)alkylene-,
-(C₁-C₄)alkylene-ethenylene-(C₀-C₂)alkylene-X-W-(C₁-C₃)alkylene-,
-(C₁-C₄)alkylene-ethynylene-(C₁-C₄)alkylene-, or
-(C₁-C₄)alkylene-ethynylene-X-(C₀-C₃)alkylene-;
W is oxy, thio, sulfino, sulfonyl, aminosulfonyl-, -mono-N-(C₁-C₄)alkyleneaminosulfonyl-, sulfonylamino, N-(C₁-C₄)alkylenesulfonylamino, carboxamido, N-(C₁-C₄)alkylenecarboxamido, carboxamidooxy, N-(C₁-C₄)alkylenecarboxamidooxy, carbamoyl, -mono-N-(C₁-C₄)alkylenecarbamoyl, carbamoyloxy, or -mono-N-(C₁-C₄)alkylenecarbamoyloxy, wherein said W alkyl groups are optionally substituted on carbon with one to three fluorines;
X is a five- or six-membered aromatic ring optionally having one or two heteroatoms selected independently from oxygen, nitrogen, and sulfur; said ring optionally mono-, or di-substituted independently with halo, (C₁-C₃)alkyl, trifluoromethyl, trifluoromethyloxy, difluoromethyloxy, hydroxyl, (C₁-C₄)alkoxy, or carbamoyl;
Z is carboxyl, (C₁-C₆)alkoxycarbonyl, tetrazolyl, 1,2,4-oxadiazolyl, 5-oxo-1,2,4-oxadiazolyl, (C₁-C₄)alkylsulfonylcarbamoyl or phenylsulfonylcarbamoyl;
K is a bond, (C₁-C₈)alkylene, thio(C₁-C₄)alkylene or oxy(C₁-C₄)alkylene, said (C₁-C₈)alkylene optionally mono-unsaturated and wherein K is optionally mono-, di- or tri-substituted independently with fluoro, methyl or chloro;
M is -Ar, -Ar¹-V-Ar², -Ar¹-S-Ar² or -Ar¹-O-Ar² wherein Ar, Ar¹ and Ar² are each independently a partially saturated, fully saturated or fully unsaturated five- to eight-membered ring optionally having one to four heteroatoms selected independently from oxygen, sulfur and nitrogen, or, a bicyclic ring consisting of two fused partially saturated, fully saturated or fully unsaturated five- or six-membered rings, taken independently, optionally having one to four heteroatoms selected independently from nitrogen, sulfur and oxygen;
said Ar, Ar¹ and Ar² moieties optionally substituted, on one ring if the moiety is monocyclic, or one or both rings if the moiety is bicyclic, on carbon with up to three substituents independently selected from R¹, R² and R³ wherein R¹, R² and R³ are hydroxy, nitro, halo, (C₁-C₆)alkoxy, (C₁-C₄)alkoxy(C₁-C₄)alkyl, (C₁-C₄)alkoxycarbonyl, (C₁-C₇)alkyl, (C₃-C₇)cycloalkyl, (C₃-C₇)cydoalkyl(C₁-C₄)alkyl, (C₃-C₇)cydoalkyl(C₁-C₄)alkanoyl, formyl, (C₁-C₈)alkanoyl; (C₁-C₅)alkanoyl(C₁-C₆)alkyl, (C₁-C₄)alkanoylamino, (C₁-C₄)alkoxycarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₅)alkylsulfinyl, (C₁-C₄)alkylsulfonyl or mono-N- or di-N,N-(C₁-C₄)alkylaminosulfinyl;
R¹, R² and R³ are optionally mono-, di- or tri-substituted on carbon independently with halo or hydroxy; and
V is a bond or (C₁-C₃)alkylene optionally mono- or di-substituted independently with hydroxy or fluoro;
(ii) a compound of Formula II 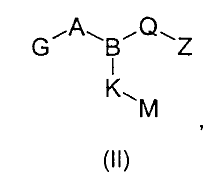 prodrugs thereof, and the pharmaceutically acceptable salts of said compounds and said prodrugs, wherein
A is SO₂ or CO;
G is Ar, Ar¹-V-Ar², Ar-(C₁-C₆)alkylene, Ar-CONH-(C₁-C₆)alkylene, R¹R²-amino, oxy(C₁-C₆)alkylene, amino substituted with Ar, or amino substituted with Ar(C₁-C₄)alkylene and R¹¹, wherein R¹¹ is H or (C₁-C₈)alkyl, R¹ and R² may be taken separately and are independently selected from H and (C₁-C₈)alkyl, or R¹ and R² are taken together with the nitrogen atom of the amino group to form a five- or six-membered azacycloalkyl, said azacycloalkyl optionally containing an oxygen atom and optionally mono-, di- or tri-substituted independently with up to two oxo, hydroxy, (C₁-C₄)alkyl, fluoro or chloro;
B is N or CH;
Q is
-(C₂-C₆)alkylene-W-(C₁-C₃)alkylene-, said alkylenes each optionally substituted with up to four substituents independently selected from fluoro or (C₁-C₄)alkyl,
-(C₄-C₈)alkylene-, said alkylene optionally substituted with up to four substituents independently selected from fluoro or (C₁-C₄)alkyl,
-X-(C₁-C₅)alkylene-, said alkylene optionally substituted with up to four substituents independently selected from fluoro or (C₁-C₄)alkyl,
-(C₁-C₅)alkylene-X-, said alkylene optionally substituted with up to four substituents independently selected from fluoro or (C₁-C₄)alkyl,
-(C₁-C₃)alkylene-X-(C₁-C₃)alkylene-, said alkylenes each optionally substituted with up to four substituents independently selected from fluoro or (C₁-C₄)alkyl,
-(C₂-C₄)alkylene-W-X-(C₀-C₃)alkylene-, said alkylenes each optionally substituted with up to four substituents each independently selected from fluoro or (C₁-C₄)alkyl,
-(C₀-C₄)alkylene-X-W-(C₁-C₃)alkylene-, said alkylenes each optionally substituted with up to four substituents each independently selected from fluoro or (C₁-C₄)alkyl,
-(C₂-C₅)alkylene-W-X-W-(C₁-C₃)alkylene-, wherein the two occurrences of W are independent of each other, said alkylenes each optionally substituted with up to four substituents each independently selected from fluoro or (C₁-C₄)alkyl,
-(C₁-C₄)alkylene-ethenylene-(C₁-C₄)alkylene-, said alkylenes and said ethenylene each optionally substituted with up to four substituents each independently selected from fluoro or (C₁-C₄)alkyl,
-(C₁-C₄)alkylene-ethenylene-(C₀-C₂)alkylene-X-(C₀-C₅)alkylene-, said alkylenes and said ethenylene each optionally substituted with up to four substituents each independently selected from fluoro or (C₁-C₄)alkyl,
-(C₁-C₄)alkylene-ethenylene-(C₀-C₂)alkylene-X-W-(C₁-C₃)alkylene-, said alkylenes and said ethenylene each optionally substituted with up to four substituents each independently selected from fluoro or (C₁-C₄)alkyl,
-(C₁-C₄)alkylene-ethynylene-(C₁-C₄)alkylene-, said alkylenes and said ethynylene each optionally substituted with up to four substituents each independently selected from fluoro or (C₁-C₄)alkyl, or
-(C₁-C₄)alkylene-ethynylene-X-(C₀-C₃)alkylene-, said alkylenes and said ethynylene each optionally substituted with up to four substituents each independently selected from fluoro or (C₁-C₄)alkyl;
Z is carboxyl, (C₁-C₆)alkoxycarbonyl, tetrazolyl, 1,2,4-oxadiazolyl, 5-oxo-1,2,4-oxadiazolyl, 5-oxo-1,2,4-thiadiazolyl, (C₁-C₄)alkylsulfonylcarbamoyl or phenylsulfonylcarbamoyl;
K is a bond, (C₁-C₉)alkylene, thio(C₁-C₄)alkylene, (C₁-C₄)alkylenethio(C₁-C₄)alkylene, (C₁-C₄)alkyleneoxy(C₁-C₄)alkylene or oxy(C₁-C₄)alkylene, said (C₁-C₉)alkylene optionally mono-unsaturated and wherein, when K is not a bond, K is optionally mono-, di- or tri-substituted independently with chloro, fluoro, hydroxy or methyl;
M is -Ar³, -Ar⁴-V¹-Ar⁵, -Ar⁴-S-Ar⁵, -Ar⁴-SO-Ar⁵, -Ar⁴-SO₂-Ar⁵ or -Ar⁴-O-Ar⁵;
Ar is a partially saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five- or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, taken independently, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur; or Ar is a fully saturated five-to seven-membered ring having one or two heteroatoms selected independently from oxygen, sulfur and nitrogen;
Ar¹ and Ar² are each independently a partially saturated, fully saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five-or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur;
said Ar, Ar¹ and Ar² moieties are optionally substituted on carbon or nitrogen, on one ring if the moiety is monocyclic, on one or both rings if the moiety is bicyclic, or on one, two or three rings if the moiety is tricyclic, with up to three substituents per moiety independently selected from R³, R⁴ and R⁵ wherein R³, R⁴ and R⁵ are independently hydroxy, nitro, halo, carboxy, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, (C₁-C₄)alkanoylamino, (C₁-C₄)alkoxycarbonylamino, hydroxysulfonyl, aminocarbonylamino or mono-N-, di-N,N-, di-N,N'- or tri-N,N,N'-(C₁-C₄)alkyl substituted aminocarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₅)alkylsulfinyl, (C₁-C₄)alkylsulfonyl or mono-N- or di-N,N-(C₁-C₄)alkylaminosulfinyl;
Ar³, Ar⁴ and Ar⁵ are each independently a partially saturated, fully saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five- or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur;
said Ar³, Ar⁴ and Ar⁵ moieties are optionally substituted on carbon or nitrogen, on one ring if the moiety is monocyclic, on one or both rings if the moiety is bicyclic, or on one, two or three rings if the moiety is tricyclic, with up to three substituents per moiety independently selected from R³¹, R⁴¹ and R⁵¹wherein R³¹, R⁴¹ and R⁵¹ are independently hydroxy, nitro, halo, carboxy, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, (C₁-C₄)alkanoylamino, (C₁-C₄)alkoxycarbonylamino, hydroxysulfonyl, aminocarbonylamino or mono-N-, di-N,N-, di-N,N'- or tri-N,N,N'-(C₁-C₄)alkyl substituted aminocarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl;
W is oxy, thio, sulfino, sulfonyl, aminosulfonyl-, -mono-N-(C₁-C₄)alkyleneaminosulfonyl-, sulfonylamino, N-(C₁-C₄)alkylenesulfonylamino, carboxamido, N-(C₁-C₄)alkylenecarboxamido, carboxamidooxy, N-(C₁-C₄)alkylenecarboxamidooxy, carbamoyl, -mono-N-(C₁-C₄)alkylenecarbamoyl, carbamoyloxy, or -mono-N-(C₁-C₄)alkylenecarbamoyloxy, wherein said W alkyl groups are optionally substituted on carbon with one to three fluorines;
X is a five- or six-membered aromatic ring optionally having one or two heteroatoms selected independently from oxygen, nitrogen, and sulfur; said ring optionally mono-, di- or tri-substituted independently with halo, (C₁-C₃)alkyl, trifluoromethyl, trifluoromethyloxy, difluoromethyloxy, hydroxyl, (C₁-C₄)alkoxy, or carbamoyl;
R¹, R², R³, R⁴ R⁵, R¹¹, R³¹, R⁴¹ and R⁵¹, when containing an alkyl, alkylene, alkenylene or alkynylene moiety, are optionally mono-, di- or tri-substituted on carbon independently with halo or hydroxy; and
V and V¹ are each independently a bond, thio(C₁-C₄)alkylene, (C₁-C₄)alkylenethio, (C₁-C₄)alkyleneoxy, oxy(C₁-C₄)alkylene or (C₁-C₃)alkylene optionally mono- or di-substituted independently with hydroxy or fluoro;
(iii) a compound of Formula III 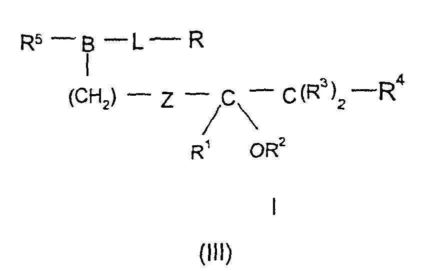 prodrugs thereof, and the pharmaceutically acceptable salts of said compounds and said prodrugs, wherein
B is N or C(Q¹), where Q¹ is H or (C₁-C₃)alkyl;
L is n-propylenyl-X- or CH₂-metaphenylene-CH₂, wherein X is furanyl, thienyl, thiazolyl or tetrahydrofuranyl, said CH₂-metaphenylene-CH₂ or X being optionally mono-, di- or tri-substituted on aromatic carbon independently with one to three chloro, fluoro, methoxy, difluoromethoxy, trifluoromethoxy, trifluoromethyl or methyl;
R is carboxyl, (C₁-C₆)alkoxycarbonyl, tetrazolyl, 5-oxo-1,2,4-thiadiazolyl; 5-oxo-1,2,4-oxadiazolyl, (C₁-C₄)alkylsulfonylcarbamoyl or phenylsulfonylcarbamoyl;
R¹ is H, methyl, ethyl or propyl;
R² is H or (C₂ - C₅) alkanoyl;
R³ is independently H, fluoro or methyl;
R⁴ is H, (C₁ - C₇) alkyl, or R⁴ and R¹ are taken together to form a 5-9 membered carbocyclic ring, said alkyl being optionally monounsaturated and optionally mono-, di- or tri-substituted independently with one to three fluoro, chloro, methoxy, difluoromethoxy, trifluoromethoxy, trifluoromethyl or methyl;
R⁵ is (C₁-C₆)alkylsulfonyl, (C₃-C₇)cycloalkylsulfonyl, (C₃-C₇)cydoalkyl(C₁-C₆)alkylsulfonyl, (C₁-C₆)alkylcarbonyl, (C₃-C₇)cycloalkylcarbonyl, (C₃-C₇)cycloalkyl(C₁-C₆)alkylcarbonyl, G-sulfonyl or G-carbonyl, said (C₁-C₆)alkylsulfonyl, (C₃-C₇)cycloalkylsulfonyl, (C₃-C₇)cycloalkyl(C₁-C₆)alkylsulfonyl, (C₁-C₆)alkylcarbonyl, (C₃-C₇)cycloalkylcarbonyl, (C₃-C₇)cycloalkyl(C₁-C₆)alkylcarbonyl optionally mono-, di- or tri- substituted on carbon independently with hydroxy, fluoro, chloro, methoxy, difluoromethoxy, trifluoromethoxy, trifluoromethyl or methyl;
Z is methylene, ethylene, propylene or ethenylene; G is Ar, Ar¹-V-Ar², Ar-(C₁-C₆)alkylene, Ar-CONH-(C₁-C6)alkylene, R¹²R¹³-amino, oxy(C₁-C₆)alkylene, amino substituted with Ar, or amino substituted with Ar(C₁-C₄)alkylene and R¹¹, wherein R¹¹ is H or (C₁-C₈)alkyl, R¹² and R¹³ may be taken separately and are independently selected from H and (C₁-C₈)alkyl, or R¹² and R¹³ are taken together with the nitrogen atom to which they are attached to form a five- or six-membered azacycloalkyl, said azacycloalkyl optionally containing an oxygen atom and optionally substituted with up to two oxo, hydroxy, (C₁-C₄)alkyl, fluoro or chloro;
Ar is a partially saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five- or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur; or Ar is a fully saturated five- to seven-membered ring having one or two heteroatoms selected independently from oxygen, sulfur and nitrogen;
Ar¹ and Ar² are each independently a partially saturated, fully saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five-or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, taken independently, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur;
said Ar, Ar¹ and Ar² moieties are optionally substituted on carbon or nitrogen, on one ring if the moiety is monocyclic, on one or both rings if the moiety is bicyclic, or on one, two or three rings if the moiety is tricyclic, with up to three substituents per moiety, independently selected from R¹⁴, R¹⁵ and R¹⁶ wherein R¹⁴, R¹⁵ and R¹⁶ are independently hydroxy, nitro, halo, carboxy, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, (C₁-C₄)alkanoylamino, (C₁-C₄)alkoxycarbonylamino, hydroxysulfonyl, aminocarbonylamino or mono-N-, di-N,N-, di-N,N'- or tri-N,N,N'-(C₁-C₄)alkyl substituted aminocarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₅)alkylsulfinyl, (C₁-C₄)alkylsulfonyl or mono-N- or di-N,N-(C₁-C₄)alkylaminosulfinyl; and
V is a bond, thio(C₁-C₄)alkylene, (C₁-C₄)alkylenethio, (C₁-C₄)alkyleneoxy, oxy(C₁-C₄)alkylene or (C₁-C₃)alkylene optionally mono- or di-substituted, when V is not a bond, independently with hydroxy or fluoro; and
(iv) a compound of Formula IV 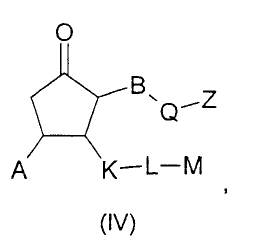 prodrugs thereof, and the pharmaceutically acceptable salts of said compounds and said prodrugs wherein
A is hydrogen or hydroxy;
B is propylene, propenylene or propynylene;
Q is propylene, -CH₂OCH₂-, thiazolyl, pyridyl, phenyl or thienyl;
Z is carboxyl, (C₁-C₆)alkoxycarbonyl, tetrazolyl, 1,2,4-oxadiazolyl or 5-oxo-1,2,4-oxadiazolyl;
K is ethylene or ethenylene;
L is a bond or -CO-;
M is -Ar, -Ar¹-V-Ar², -Ar¹-S-Ar² or -Ar¹-O-Ar² wherein
Ar and Ar¹ are either
(1) each independently a fully unsaturated five- to eight-membered ring optionally having one to four heteroatoms selected independently from oxygen, sulfur and nitrogen, or a bicyclic ring consisting of two fused partially saturated, fully saturated or fully unsaturated five- and/or six-membered rings, taken independently, optionally having one to four heteroatoms selected independently from nitrogen, sulfur and oxygen, or a tricyclic ring consisting of three fused partially saturated, fully saturated or fully unsaturated five- and/or six-membered rings, taken independently, optionally having one to four heteroatoms selected independently from nitrogen, sulfur and oxygen, any of said partially saturated or fully saturated rings optionally having one or more oxo groups substituted on carbon, or
(2) each independently a fully saturated five to eight-membered ring;
Ar² is a partially saturated, fully saturated or fully unsaturated five- to eight-membered ring optionally having one to four heteroatoms selected independently from oxygen, sulfur and nitrogen, or a bicyclic ring consisting of two fused partially saturated, fully saturated or fully unsaturated five- and/or six-membered rings, taken independently, optionally having one to four heteroatoms selected independently from nitrogen, sulfur and oxygen, or a tricyclic ring consisting of three fused partially saturated, fully saturated or fully unsaturated five- and/or six-membered rings, taken independently, optionally having one to four heteroatoms selected independently from nitrogen, sulfur and oxygen, any of said partially saturated or fully saturated rings optionally having one or more oxo groups substituted on carbon;
said Ar and Ar¹ moieties, when a fully unsaturated five- to eight-membered ring, a bicyclic ring or a tricyclic ring, and said Ar² moieties are each independently optionally substituted on carbon, on one ring if the moiety is monocyclic, on one or both rings if the moiety is bicyclic, or on one, two or three rings if the moiety is tricyclic, with up to three substituents selected from R¹, R² and R³ wherein R¹, R² and R³ are independently hydroxy, nitro, halo, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, aminocarbonylamino or mono-N-, di-N,N-, di-N,N'- or tri-N,N,N'-(C₁-C₄)alkyl substituted aminocarbonylamino, (C₁-C₄)alkanoylamino, (C₁-C₄)alkoxycarbonylamino, sulfonamido, hydroxysulfonyl, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl;
R¹, R² and R³, when containing an alkyl, alkenyl, alkylene or alkenylene moiety, are optionally straight or branched and are optionally mono-, di- or tri-substituted on carbon independently with halo or hydroxy; and
V is a bond, -CO- or (C₁-C₃)alkylene optionally mono- or di-substituted independently with hydroxy or fluoro.

2. The use according to claim 1 wherein the compound is a compound of Formula 1, a prodrug thereof, or a pharmaceutically acceptable salt of siad compound, or said prodrug.

3. The use according to claim 2 wherein the compound is selected from:
7-[(2'-hydroxymethyl-biphenyl-4-ylmethyl)-methanesulfonyl-amino]-heptanoic acid;
7-{[4-(3-hydroxymethyl-thiophen-2-yl)-benzyl]-methanesulfonyl-amino}-heptanoic acid;
7-[(2'-chloro-biphenyl-4-ylmethyl)-methanesulfonyl-amino]-heptanoic acid;
7-{[4-(1-hydroxy-hexyl)-benzyl]-methanesulfonyl-amino}-heptanoic acid;
7-[(4-butyl-benzyl)-methanesulfonyl-amino]-heptanoic acid;
7-{[5-(1-hydroxy-hexyl)-thiophen-2-ylmethyl]-methanesulfonyl-amino}-heptanoic acid;
(3-{[(4-butyl-benzyl)-methanesufonyl-amino]-methyl}-phenyl)-acetic acid;
7-{[3-(3-chloro-phenyl)-propyl]-methanesulfonyl-amino}-heptanoic acid;
7-{[3-(3,5-dichloro-phenyl)-propyl]-methanesufonyl-amino}-heptanoic acid;
5-(3-{[3-(3-chloro-phenyl)-propyl]-methanesulfonyl-amino}-propyl)-thiophene-2-carboxylic acid;
7-{[2-(3,5-dichloro-phenoxy)-ethyl]-methanesulfonyl-amino}-heptanoic acid;
5-(3-{[2-(3,5-dichloro-phenoxy)-ethyl]-methanesulfonyl-amino}-propyl)-thiophene-2-carboxylic acid;
N-[2-(3,5-dichloro-phenoxy)-ethyl]-N-[6-(1H-tetrazol-5-yl)-hexyl]-methanesulfonamide;
*trans*-(4-{[3-(3,5-dichloro-phenyl)-allyl]-methanesulfonyl-amino}-butoxy)-acetic acid;
*trans*-N-[3-(3,5-dichloro-phenyl)-allyl]-N-[6-(1H-tetrazol-5-yl)-hexyl]-methanesulfonamide;
*trans*-5-(3-{[3-(3,5-dichloro-phenyl)-allyl]-methanesulfonyl-amino}-propyl)-thiophene-2-carboxylic acid; and
*trans*-[3-({[3-(3,5-dichloro-phenyl)-allyl]-methanesulfonyl-amino}-methyl)-phenyl]-acetic acid;
prodrugs thereof, and the pharmaceutically acceptable salts of said compounds and said prodrugs.

4. The use according to claim 1 wherein the compound is a compound of Formula II, a prodrug thereof, or a pharmaceutically acceptable salt of said compound, or said prodrug.

5. The use according to claim 4 wherein the compound is selected from:
(3-(((pyridine-3-sulfonyl)-(4-pyrimidin-5-yl-benzyl)-amino)-methyl)-phenyl)-acetic acid;
(3-(((5-phenyl-furan-2-ylmethyl)-(pyridine-3-sulfonyl)-amino)-methyl)-phenyl)-acetic acid;
(3-(((pyridine-3-sulfonyl)-(4-pyrimidin-2-yl-benzyl)-amino)-methyl)-phenyl)-acetic acid;
(3-(((pyridine-3-sulfonyl)-(4-thiazol-2-yl-benzyl)-amino)-methyl)-phenyl)-acetic acid;
(3-(((4-pyrazin-2-yl-benzyl)-(pyridine-3-sulfonyl)-amino)-methyl)-phenyl)-acetic acid;
(3-(((4-cyclohexyl-benzyl)-(pyridine-3-sulfonyl)-amino)-methyl)-phenoxy)-acetic acid;
(3-(((pyridine-3-sulfonyl)-(4-pyridin-2-yl-benzyl)-amino)-methyl)-phenoxy)-acetic acid;
(3-(((pyridine-3-sulfonyl)-(4-pyridin-3-yl-benzyl)-amino)-methyl)-phenoxy)-acetic acid;
(3-(((pyridine-3-sulfonyl)-(4-pyridin-4-yl)-benzyl)-amino)-methyl)-phenoxy)-acetic acid;
(3-(((pyridine-3-sulfonyl)-(4-thiazol-2-yl-benzyl)-amino)-methyl)-phenoxy)-acetic acid;
(3-(((2,3-dihydro-benzo[1,4]dioxin-6-ylmethyl)-(pyridine-3-sulfonyl)-amino)-methyl)-phenyl)-acetic acid;
(3-(((benzofuran-2-ylmethyl-(pyridine-3-sulfonyl)-amino)-methyl)-phenyl)-acetic acid;
(3-(((4-butyl-benzyl)-(pyridine-3-sulfonyl)-amino)-methyl}-phenyl)-acetic acid;
(3-(((benzenesulfonyl-(4-butyl-benzyl)-amino)-methyl)-phenyl)-acetic acid;
(3-(((4-butyl-benzyl)-(1-methyl-1 H-imidazole-4-sulfonyl)-amino)-methyl)-phenyl)-acetic acid;
(3-(((4-dimethylamino-benzyl)-(pyridine-3-sulfonyl)-amino)-methyl)-phenyl)-acetic acid;
(3-(((4-dimethylamino-benzyl)-(pyridine-3-sulfonyl)-amino)-methyl)-phenoxy)-acetic acid;
(3-(((4-tert-butyl-benzyl)-(pyridine-3-sulfonyl)-amino)-methyl)-phenoxy)-acetic acid;
*trans*-(3-(((3-(3,5-dichloro-phenyl)-allyl)-(pyridine-3-sulfonyl)-amino)-methyl)-phenyl)-acetic acid; and
(3-(((2-(3,5-dichloro-phenoxy)-ethyl)-(pyridine-3-sulfonyl)-amino)-methyl)-phenoxy)-acetic acid;
prodrugs thereof, and the pharmaceutically acceptable salts of said compounds, and said prodrugs.

6. The use according to claim 1 wherein the compound is a compound of Formula III, a prodrug thereof, or a pharmaceutically acceptable salt of said compound, or said prodrug.

7. The use according to claim 6 wherein B is N; R is carboxyl, (C₁-C₆)alkoxycarbonyl or tetrazolyl; Z is ethylenyl; R¹ and R² are each H; and L is CH₂-metaphenylene-CH₂ or n-propylene-X-; a prodrug thereof, or a pharmaceutically acceptable salt of said compound, or said prodrug.

8. The use according to claim 7 wherein R⁵ is selected from (C₁-C₆)alkycarbonyl, optionally mono-, di-, or tri-substituted with hydroxy or fluoro; (C₁-C₃)alkysulfonyl or (C₃-C₇)cycloalkysulfonyl; and G-sulfonyl, wherein G is phenyl, imidazolyl, pyridyl, pyrazolyl, or pyrimidyl optionally mono-, di-, or tri-substituted on carbon or nitrogen with chloro, fluoro, methoxy, difluoromethoxy, trifluoromethoxy, trifluoromethyl or methyl; a prodrug thereof, or a pharmaceutically acceptable salt of said compound, or said prodrug.

9. The use according to claim 1 wherein the compound is a compound of Formula IV, a prodrug thereof, or a pharmaceutically acceptable salt of said compound, or said prodrug.

10. The use according to claim 9 wherein the compound is selected from:
*trans*-7-(2-(2-(3,5-bis-trifluoromethyl-phenyl)-vinyl)-5-oxo-cyclopentyl)-heptanoic acid;
*trans*-7-(2-(2-(4-chloro-3-trifluoromethyl-phenyl)-vinyl)-5-oxo-cyclcopentyl)-heptanoic acid;
*trans*-7-(2-(2--(3,5-dichlorophenyl)-vinyl-5-oxo-cyclopentyl)-heptanoic acid;
*trans*-7-(2-(2-(3-chlorophenyl-vinyl)-5-oxo-cyclopentyl)-heptanoic acid;
*trans*-7-(2-oxo-5-(2-(3-trifluoromethyl-phenyl)-vinyl)-cyclopentyl)-heptanoic acid;
*trans*-7-(2-(2-(4-fluoro-phenyl)-vinyl)-5-oxo-cyclopentyl)-heptanoic acid;
ethyl *trans*-7-(2-(2-(3,5-bis-trifluoromethyl-phenyl)-vinyl)-5-oxocyclopentyl)-heptanoate;
ethyl *trans*-7-(2-(2-(4-chloro-3-trifluoromethyl-phenyl)-vinyl)-5-oxo-cyclopentyl)-heptanoate;
ethyl *trans*-7-(2-(2-(3,5-dichlorophenyl)-vinyl)-5-oxo-cyclopentyl)-heptanoate;
ethyl *trans*-7-(2-(2-(3-chlorophenyl)-vinyl)-5-oxo-cyclopentyl)-heptanoate;
ethyl *trans*-7-(2-oxo-5-(2-(3-trifluoromethyl-phenyl)-vinyl)-cyclopentyl)-heptanoate;
ethyl *trans*-7-(2-(2-(4-fluoro-phenyl)-vinyl)-5-oxo-cyclopentyl)-heptanoate;
*trans*-3-(2-(3,5-bis-trifluoromethyl-phenyl)-vinyl)-2-(6-(2H-tetrazol-5-yl)-hexyl)-cyclopentanone;
*trans*-3-(2-(4-chloro-3-trifluoromethylphenyl)-vinyl)-2-(6-(2H-tetrazol-5-yl)-hexyl)-cyclopentanone;
*trans*-3-(2-(3,5-dichloro-phenyl)-vinyl)-2-(6-(2H-tetrazol-5-yl)-hexyl)-cyclopentanone;
*trans*-3-(2-(3-chloro-phenyl)-vinyl)-2-(6-(2H-tetrazol-5-yl)-hexyl)-cyclopentanone;
*trans*-3-(2-(3-trifluoromethyl-phenyl)-vinyl)-2-(6-(2H-tetrazol-5-yl)-hexyl)-cyclopentanone; and
*trans*-3-(2-(4-fluoro-phenyl)-vinyl)-2-(6-(2H-tetrazol-5-yl)-hexyl)-cyclopentanone;
prodrugs thereof, and the pharmaceutically acceptable salts of said compounds, and said prodrugs.

11. The use according to claim 1 wherein the compound is administered orally.

12. The use according to claim 1 wherein the compound is administered transdermally.
